# EUROPEAN PATENT SPECIFICATION

(11) **EP 0 341 209 B1**
(45) Date of publication and mention of the grant of the patent: **15.09.1993**
(21) Application number: 89810317.1
(22) Date of filing: 27.04.1989
(51) Int. Cl.: A61K 35/12

(54) **Physiologically active substances, a process for preparation and pharmaceutical compositions thereof**
Physiologisch wirkende Substanzen, Verfahren zu ihrer Herstellung und pharmazeutische Zusammensetzungen davon
Substances physiologiquement activées, procédé pour leur préparation et leurs composés pharmaceutiques

(30) Priority: 30.04.1988 JP 108590/88
(43) Date of publication of application: 08.11.1989
(73) Proprietor: NIPPON ZOKI PHARMACEUTICAL CO. LTD., Osaka (JP)
(72) Inventor: Konishi, Jin-emon c/o Inst. of Bio-Active Science, Yashiro-Cho Katoh-gun Hyogo (JP); Hamada, Giichi c/o Hirano Factory, Hiranohonmachi, Hirano-ku Osaka (JP)
(74) Representative: Kerr, Andrew

(56) References cited:
- EP-A- 0 300 973
- EP-A- 0 315 591
- CHEMICAL ABSTRACTS, vol. 98, 1983, page 383, abstract no. 221826z, Columbus, Ohio, US
- CHEMICAL ABSTRACTS, vol. 101, 1984, page 436, abstract no. 52735s, Columbus, Ohio, US; S. AONUMA et al.: "Studies on anti-ulcerogenic protein in inflamed rabbit skin tissues. III. Anti-ulcerogenic peptide (Gly-Ser-His-Lys) obtained from tissues infected with vaccinia virus"

## Description

The present invention relates to physiologically active substances extracted from infected tissue, a process for the preparation, pharmaceutical compositions and use thereof.

As a result of investigations for physiologically active substances extracted from infected tissues and produced by inoculation with a poxvirus to animal tissues, organs or cultured cells, the novel substances extracted from infected tissues have been found having excellent pharmacological actions such as analgesic, sedative and anti-inflammatory actions, especially on diseased animals, for example, animals under stresses.

Oral drugs are easy to be taken for patients and preferable to other preparations such as injections. But many drugs do not exhibit their effects at oral administrations. It has been elucidated that the physiologically active substances of the present invention show evident therapeutic effects at both oral and parenteral administrations.

An object of the present invention is to provide a process for the production of a substance which is physiologically active as a sedative and analgesic, characterized in that
i. tissue, organs or cultured cells obtained from an animal infected with a poxvirus is homogenised with an aqueous extraction medium,
ii. the resulting extract is subjected to deproteinization,
iii. the deproteinized extract is adsorbed on a adsorbent,
iv. the adsorbed material is eluted with a basic aqueous elutant solution,
v. the eluted product in aqueous solution is treated with ethanol and the precipitate collected
vi. the eluted material is fractionated to obtain a molecular weight fraction of less than 10,000.

Another object of the invention is to provide a physiologically active substance suitable for use as a sedative or as an analgesic for the relief of chronic pain obtainable by the process described above.

A further object of the invention is to provide a pharmaceutical composition comprising the physiologically active substances as an active ingredient.

A further object of the invention is to provide the use of the physiologically active substances for the preparation of a composition suitable for use as a sedative or as an analgesic for the relief of chronic pain.

The term "infected tissues" as used in this specification is defined as meaning: animal tissues, organs or cultured cells inoculated or infected with a poxvirus.

A poxvirus, for example, orthopoxvirus such as vaccinia virus, coxpox virus, variola virus, infectious ectromelia virus or monkeypox virus, parapoxvirus such as orf virus, paravaccinia virus or bovine papular stomatitis virus or lumpy skin disease virus, avipoxvirus such as fowlpox virus or hare fibroma virus, leporipoxvirus such as rabbit myxoma virus or rabbit fibroma virus, swinepoxvirus, Yaba monkey tumor virus or Tarapox virus, can be used.

To obtain the infected tissues, various kinds of animals or birds can be utilized, for example, rabbit, sheep, goat, pig, cow, horse, monkey, hamster, guinea pig, rat, mouse or hen can be employed. The animal or bird can be selected according to a specie of poxvirus and other conditions. Also any kind of cultured cell, in which the selected poxvirus can multiply, is available, for example, cultured cell or tumor cell of kidney, skin, lung, testis, liver, muscle, adrenal, thyroid gland, brain, nerve cell or blood cell of rabbit, sheep, goat, pig, cow, horse, monkey, hamster, guinea pig, rat, mouse, hen or their embryo, cultured cell derived from human such as Hela cell, or decidua of the hatching egg can be employed.

The infected tissues are collected under aseptic conditions and ground to as small a size as possible. An extraction medium is added to the ground material which is then homogenized. As an extraction medium, distilled water, physiological saline, weakly acidic or basic buffer may be used, and if desired, a stabilizer such as glycerin, a disinfectant or preservative such as phenol, or an inorganic salt such as sodium chloride, potassium chloride or magnesium chloride can be added to the medium. At that time, the extraction can be facilitated by a procedure to disintegrate cell tissues, such as freeze-thaw extraction, sonication or treatment with a detergent or an enzyme dissolving cell membrane.

The resulting emulsion is filtered or centrifuged to remove tissue fragments. The filtrate or supernatant is deproteinized which can be carried out according to a known method, for example, heating, sonication, treatment with a protein-denaturant such as an acid, a base, urea, guanidine, an organic solvent or a detergent, iso-electric point precipitation or salting-out technique. Subsequently, the denatured proteins thereby precipitated are removed by filtration using a filter paper such as cellulose or nitrocellulose, a glass filter, sellaite and Seitz's filter , ultrafiltration, gel filtration, ion-exchange chromatography or centrifugation.

The resulting extract containing the active substances is acidified, preferably to pH 3.5 - 5.5, by addition of an acid such as hydrochloric acid, sulfuric acid or hydrobromic acid, and then subjected to adsorption to an adsorbent such as active carbon, kaolin or an ion-exchange resin. The adsorbent can be added to the extracted solution and it is stirred, or the extracted solution can be passed through a column of the adsorbent.

To elute the material containing the active substances of the present invention, a basic solution is added to the absorbent, preferably adjusting the suspension to pH 9 - 12, and then the mixture is incubated or stirred at room temperature or at a suitable temperature above room temperature by heating. The elution is achieved by removing the absorbent according to a known method such as filtration or centrifugation.

The eluate thus obtained is concentrated, or concentrated to dryness and dissolved in water. The aqueous solution of eluted material is added to ethanol to be from 60% to 95%, preferably from 70% to 90% of ethanol concentration. The precipitated material is collected by a conventional way such as filtration or centrifugation.

After the collected precipitate is dissolved in water, fractionation based on molecular weight such as ultrafiltration or gel filtration is carried out to give the active substances of the present invention.

The following examples, describe the preparation of the physiologically active substances of the present invention.

### Example 1

Vaccinia virus was inoculated to the skin of healthy adult rabbit. The inflamed skin was cut off under aseptic conditions and well ground. Aqueous phenol solution was added to this ground material and subjected to homogenization, and the emulsion was filtered by centrifugation. The resulting filtrate was adjusted to pH 4.8 - 5.5, and then heated in a stream of 100°C steam. After removing proteins thereby precipitated by filtration, the filtrate was adjusted to about pH 9.0 by addition of sodium hydroxide, heated at 100°C and filtered. The filtrate was adjusted to pH 3.5 - 5.0 and 7.5% - 15% active carbon was added thereto. After stirring for 3 - 5 hrs, the suspension was filtered. Water was added to the resulting active carbon and the suspension was adjusted to pH 10.5 - 11.5 by addition of sodium hydroxide. The extraction procedure was carried out by stirring for 1.5 - 3.0 hrs at 45 - 60°C. The suspension was filtered to remove the active carbon. The filtrate was adjusted to near neutral pH by addition of hydrochloric acid and concentrated to dryness under reduced pressure. The yield of the product was 4 - 6 g from 1 kg of infected tissues.

### Example 2

The product obtained in Example 1 was dissolved in distilled water. The aqueous solution was added to about 2.5 to 9 times volume of ethanol and the precipitate was collected by filtration. The collected precipitate was dissolved in distilled water and any insoluble matter was removed by filtration. Subsequently, the solution was filtered through a ultrafiltration membrane eliminating substances molecular weight of which is more than 10,000. To the remaining solution concentrated to small amount, distilled water was added and it was filtrated again. The procedure was repeated several times. As the resulting filtrate, the physiologically active substances of the present invention were obtained.

The substances of the invention thus obtained was concentrated to dryness under reduced pressure. As a result of weighing the dried substances, the yield of the substances of the present invention is 1.0 - 1.5 g when 1 kg of infected skin-tissues of mature rabbit are employed.

The physiologically active substances obtained in the above examples is pale yellowish brown and hygroscopic powder with a slightly peculiar smell; having a molecular weight of less than 10,000; having a UV adsorption maximum at 265 - 275 nm; comprising amino acids such as tyrosine, phenylalanine and alanine, organic acids such as urocanic acid, lactic acid and oxalic acid, bases of nucleic acid such as adenine, thymine, guanine and cytosine, sugar, uracil, xanthine, phosphorus; being negative on various methods of protein analysis; being stable for digestive enzymes such as pepsin, trypsin, chymotrypsin, amylase and lipase; and having no antigenicity.

The following descriptions serve to illustrative pharmaceutical studies of the substances of the present invention.

### I. Toxicity test

The physiologically active substances of the present invention were administered to groups each consisting of 10 SD-JCL strain female rats, ddy strain female mice and Japanese white rabbits orally. LD₅₀ of the substances of this invention were more than 10,000 mg/kg independently of species of animals and differences between the sexes. It was found that the substances of the present invention had very low toxicity.

As a result of subacute toxicity tests, no abnormality was observed at any organs. The reproduction tests showed no effect on pregnant animal, fetus, newborn and reproductivity of offspring (F₁).

### II. Pharmacological study

### (1) Analgesic effect

Analgesic effect of the substances of the present invention was studied by analgesic test methods such as Acetic acid method and Randall-Selitto method.

Acetic acid method: after intraperitoneal injection of acetic acid in physiological saline to mice, the frequency of writhing was counted.

Randall-Selitto method: pressure was applied to the tail of mice and the analgesic coefficients are determined by the minimum pressure by which animals started to escape.

ED₅₀ value of the substances of the present invention in SART-stressed animal [Folia Pharmacol. Jap.; 71, 195-210 (1975)] was from one third to two fifth of the value in experiments using normal animals. The substances of the present invention showed more remarkable effect shown in analgesic tests using SART-stressed animals than in experiments using normal animals. Furthermore, SART-stressed animals showed significant lowering of the pain thresholds compared with the normal animals, i.e. they are falling into hyperalgesic state. In SART-stressed animals the substances of the present invention evidently restored the pain threshold to normal level.

### (2) Sedative effect

Significant supression of spontaneous motor activity and exploratory movement was observed in mice administered with the substances of the invention in comparison to a control group.

### (3) Clinical study

Comparative clinical studies were carried out between drugs on the market and the pharmaceutical composition containing the substances of the present invention as an active ingredient. Therapeutic effects of the pharmaceutical composition of the present invention in the treatment of low back pain were compared with Ketoprofen. 16 mg daily of the active ingredient as the substances of the present invention was orally administered for 2 weeks to patient suffering from low back pain. In the case of Ketoprofen, 150 mg daily was administered in the same manner. Improvement rate of the group of the pharmaceutical composition of the present invention was equal to that of the Ketoprofen group, but safety of this pharmaceutical composition was significantly superior to that of Ketoprofen.

Also the comparative test with Indomethacin was done in osteoarthritis of the knee. Equal dosage of the physiologically active substances of the invention as mentioned above and 75 mg daily of Indomethacin were orally administered for 4 weeks to patents suffering from osteoarthritis of the knee. Improvement rate of the group of the pharmaceutical composition of the present invention was equal to that of the Ketoprofen group, but with respect to safety the present composition was clearly superior to Indomethacin.

As shown by the above-mentioned results, the physiologically active substances of the present invention have excellent therapeutic effects on dolorous diseases such as neuralgia, osteoarthritis of knee, low back pain, articular rheumatism and pain of gout, especially chronic symptoms of these diseases.

The above pharmacological studies using the animal models for human disease, such as stressed animals, showed that the substances of the present invention have a characteristic effect improving the abnormal hyperalgesic state under the conditions of the lowered pain threshold, and restoring the pain threshold to normal level. Namely the substances of this invention show remarkable effects especially on diseased animals which are not in a normal state.

The physiologically active substances of the present invention have therapeutic effects at even oral administration as well as have low toxicity and great safety compared with drugs on the market such as Ketoprofen and Indomethacin. Therefore, the substances of the invention can be used for long-term continuous administrations and so have the advantage of treatment for a chronic diseases.

The substances of the present invention can be made into pharmaceutical compositions by combination with appropriate medicinal carriers or diluents, and can be formulated into preparations in solid or liquid form such as tablets, capsules, powders, granules, syrups, injections or suppositories in usual ways for oral or parenteral administrations.

In pharmaceutical dosage forms, the substances of the present invention can be used in the form of their pharmaceutically acceptable salts, and also can be used alone or in appropriate association, as well as in combination with other pharmaceutically active components.

In case of oral preparations, the substances can be used alone or combined with appropriate additives to make tablets, capsules, powders, granules or syrups, e.g. with conventional additives such as lactose, mannitol, corn starch or potato starch; with binders such as crystalline cellulose, cellulose derivatives, acacia, corn starch or gelatins; with disintegrators such as corn starch, potato starch or calcium carboxymethylcellulose; with lubricants such as talc or magnesium stearate; and if desired, with diluents, buffering agents, moistening agents, preservatives and flavoring agents.

The substances of the present invention can be formulated into a preparations for injections or suppositories according to the conventional method.

The desirable dose of the substances of the present invention varies with the subject, form of the drug, method and period of administration. However, in order to obtain desirable effects, generally it is recommended to administer orally 1 to 100 mg, preferably 4 to 40 mg daily for an adult.

In case of parenteral administrations such as injections, doses of the substances in the order of one tenth to one third of the above dose are preferable as daily doses.

A prescription of the pharmaceutical compositions is shown below as one of examples which contain the substances of the present invention as an active ingredient.

| Prescription example 1 (tablet) | |
|---|---|
| Component | Content in a tablet (mg) |
| substances of this invention | 4 |
| lactose | 106 |
| crystalline cellulose | 40 |
| calcium carboxymethylcellulose | 20 |
| magnesium stearate | 10 |
| | Total 180 mg |

## Claims

1. A process for the production of a substance which is physiologically active as a sedative and analgesic, characterised in that
i. tissue, organs or cultured cells obtained from an animal infected with a poxvirus is homogenised with an aqueous extraction medium,
ii. the resulting extract is subjected to deproteinization,
iii. the deproteinized extract is adsorbed on an adsorbent,
iv. the adsorbed material is eluted with a basic aqueous elutant solution,
v. the eluted product in aqueous solution is treated with ethanol and the precipitate collected
vi. the eluted material is fractionated to obtain a molecular weight fraction of less than 10,000.

2. A process according to claim 1 wherein the poxvirus is vaccinia virus.

3. A process according to claim 1 or claim 2 wherein infected tissue from rabbit skins is utilised.

4. A process according to any preceding claim wherein the deproteinized extract is adjusted to pH 3.5 - 5.5 prior to adsorption.

5. A process according to any preceding claim wherein the elution is carried out at pH 9 - 12.

6. A process according to any preceding claim wherein the eluted material is subjected to ultrafiltration.

7. A process according to any preceding claim wherein the adsorbent is selected from kaolin and an ion-exchange resin.

8. A process according to any preceding claim wherein the adsorbent is active carbon.

9. A physiologically active substance suitable for use as a sedative or as an analgesic for the relief of chronic pain obtainable by the process of any preceding claim.

10. A substance according to claim 9 characterised by the following properties:
Appearance: Pale yellowish brown hygroscopic powder with a slightly peculiar smell
Molecular weight: less than 10,000
Ultraviolet absorption: 1 max = 265 - 275 nm.
comprising amino acids including tyrosine, phenylalanine and alanine, organic acids including urocanic acid, lactic acid and oxalic acid; bases of nucleic acids including adenine, thymine, guanine and cytosine; sugar, uracil, xanthine and phosphorus, the substance being negative to protein analysis, stable to digestive enzymes including pepsin, trypsin, chymotrypsin, amylase and lipase and having no antigenicity.

11. A pharmaceutical composition comprising as an active ingredient a substance according to claim 9 or claim 10.

12. Use for the preparation of a composition suitable for use as a sedative or as an analgesic for the relief of chronic pain of a substance according to claim 9 or claim 10.

13. Use according to claim 12 wherein said composition is suitable for use as an analgesic in the treatment of neuralgia, osteoarthritis of the knee, low back pain, articular rheumatism and gout.

14. Use according to claim 12 wherein said composition is active in improving abnormal hyperalgesia under conditions of lowered pain threshold and restoring the pain threshold to normal level.

## Patentansprüche

1. Verfahren zur Herstellung einer Substanz, welche physiologisch wirksam ist als ein Sedativum und Analgetikum, dadurch gekennzeichnet dass,
i. Gewebe, Organe oder Kulturzellen, die aus einem mit einem Pockenvirus infizierten Tier erhalten wurden, mit einem wässrigen Extraktionsmedium homogenisiert werden,
ii. der resultierende Extrakt Deproteinisierung unterworfen wird,
iii. der deproteinisierte Extrakt an ein Adsorbens adsorbiert wird,
iv. das adsorbierte Material mit einer basischen wässrigen Elutionslösung eluiert wird,
v. das eluierte Produkt in wässriger Lösung mit Äthanol behandelt wird und das Präzipitat gesammelt wird,
vi. das eluierte Material fraktioniert wird, um eine Fraktion mit einem Molekulargewicht von weniger als 10'000 zu erhalten.

2. Verfahren nach Anspruch 1 worin der Pockenvirus ein Vacciniavirus ist.

3. Verfahren nach Anspruch 1 oder Anspruch 2, worin infiziertes Gewebe aus Kaninchenhaut verwendet wird.

4. Verfahren nach einem der vorangehenden Ansprüche, worin der deproteinisierte Extrakt vor Adsorption auf pH 3,5 - 5,5 eingestellt wird.

5. Verfahren nach einem der vorangehenden Ansprüche, worin die Elution bei pH 9 - 12 ausgeführt wird.

6. Verfahren nach einem der vorangehenden Ansprüche, worin das eluierte Material Ultrafiltration unterworfen wird.

7. Verfahren nach einem der vorangehenden Ansprüche, worin das Adsorbens aus Kaolin und einem Ionenaustauscherharz ausgewählt wurde.

8. Verfahren nach einem der vorangehenden Ansprüche, worin das Adsorbens Aktivkohle ist.

9. Physiologisch wirksame Substanz, geeignet für die Verwendung als ein Sedativum oder als ein Analgetikum zur Linderung von chronischen Schmerzen, die durch das Verfahren irgendeines vorangehenden Anspruchs erhältlich ist.

10. Substanz nach Anspruch 9, gekennzeichnet durch die folgenden Eigenschaften:
Aussehen: blassgelblichbraunes hygroskopisches Pulver mit einem leicht merkwürdigem Geruch
Molkulargewicht: weniger als 10'000
Ultraviolettabsorption: λmax = 265 - 275 nm,
umfassend Aminosäuren einschliesslich Tyrosin, Phenylalanin und Alanin, organische Säuren einschliesslich Urocaninsäure, Milchsäure und Oxalsäure; Aminosäurebasen einschliesslich Adenin, Thymin, Guanin und Cytosin; Zucker, Uracil, Xanthin und Phosphor, wobei die Sustanz bei verschiedenen Proteinanalyseverfahren negative Resultate ergibt, stabil gegenüber Verdauungsenzymen einschliesslich Pepsin, Trypsin, Chymotrypsin, Amylase und Lipase ist und keine antigene Potenz hat.

11. Pharmazeutische Zusammensetzung, die als Wirkstoff eine Substanz nach Anspruch 9 oder Anspruch 10 enthält.

12. Verwendung einer Substanz nach Anspruch 9 oder 10 für die Herstellung einer Zusammensetzung, die geeignet ist für die Verwendung als Sedativum oder als Analgetikum für die Linderung von chronischen Schmerzen.

13. Verwendung nach Anspruch 12, worin besagte Zusammensetzung geeignet für die Verwendung als Analgetikum in der Behandlung von Neuralgie, Osteoarthritis des Knies, Kreuzschmerzen, Gelenkrheumatismus und Gicht ist.

14. Verwendung nach Anspruch 12, worin besagte Zusammensetzung bei der Verbesserung der anomalen Hyperalgesie bei Zuständen verminderter Schmerzgrenze wirksam ist und die Schmerzgrenze auf das normale Niveau zurückbringt.

## Revendications

1. Un procédé pour la production d'une substance qui est physiologiquement active comme sédatif et analgésique, caractérisé en ce que :
i) on homogénéise avec un milieu d'extraction aqueux des tissus, organes ou cellules de culture obtenus à partir d'un animal infecté par un poxvirus,
ii) on soumet à une déprotéinisation l'extrait résultant,
iii) on adsorbe sur un adsorbant l'extrait déprotéinisé,
iv) on élue la matière adsorbée par une solution éluante aqueuse basique,
v) on traite à l'éthanol le produit élué en solution aqueuse et on recueille le précipité, et
vi) on fractionne la matière éluée de manière à obtenir une fraction de poids moléculaire inférieur à 10 000.

2. Un procédé selon la revendication 1, dans lequel le poxvirus est le virus de la vaccine.

3. Un procédé selon la revendication 1 ou 2, dans lequel on utilise un tissu infecté provenant de peaux de lapin.

4. Un procédé selon l'une des revendications précédentes, dans lequel on ajuste avant adsorption le pH de l'extrait déprotéinisé à une valeur comprise entre 3,5 et 5,5.

5. Un procédé selon l'une des revendications précédentes, dans lequel on exécute l'élution à un pH compris entre 9 et 12.

6. Un procédé selon l'une des revendications précédentes, dans lequel on soumet le matériau élué à une ultrafiltration.

7. Un procédé selon l'une des revendications précédentes, dans lequel on choisit l'adsorbant parmi le kaolin et une résine échangeuse d'ions.

8. Un procédé selon l'une des revendications précédentes, dans lequel l'adsorbant est le charbon actif.

9. Une substance physiologiquement active, convenant à une utilisation comme sédatif ou comme analgésique pour le soulagement des douleurs chroniques, pouvant être obtenue par le procédé de l'une des revendications précédentes.

10. Une substance selon la revendication 9, caractérisée par les propriétés suivantes :
Aspect : poudre hygroscopique brun-jaunâtre, pâle avec une légère odeur particulière,
Poids moléculaire : inférieur à 10 000,
Absorption UV : λₘₐₓ = 265-275 nm,
comprenant des acides aminés tels que la tyrosine, la phénylalanine et l'alanine, des acides organiques tels que l'acide urocanique, l'acide lactique et l'acide oxalique, des bases d'acides nucléiques telles que l'adénine, la thymine, la guanine et la cytosine, du sucre, de l'uracile, de la xanthine, du phosphore, ladite substance réagissant négativement à l'analyse des protéines, étant stable aux enzymes digestives telles que la pepsine, la trypsine, la chymotrypsine, l'amylase et la lipase et ne présentant pas d'antigénicité.

11. Une composition pharmaceutique, comprenant comme principe actif une substance selon la revendication 9 ou 10.

12. Une uutilisation d'une substance selon la revendication 9 ou 10 pour la préparation d'une composition convenant à une utilisation comme sédatif ou comme analgésique pour le soulagement des douleurs chroniques.

13. L'utilisation selon la revendication 12, dans laquelle ladite composition convient à une utilisation comme analgésique dans le traitement des névralgies, de l'ostéoarthrite du genou, des douleurs du bas du dos, des rhumatismes articulaires et de la goutte.

14. L'utilisation selon la revendication 12, dans laquelle ladite composition est active pour améliorer l'hyperalgésie normale dans des conditions d'abaissement du seuil de la douleur et pour restaurer le seuil de la douleur à un niveau normal.
